# EUROPÄISCHE PATENTSCHRIFT

(11) **EP 3 429 514 B1**
(45) Veröffentlichungstag und Bekanntmachung des Hinweises auf die Patenterteilung: **11.08.2021**
(21) Anmeldenummer: 17710276.1
(22) Anmeldetag: 13.03.2017
(51) Int. Cl.: A61F 5/01

(54) **MEDIZINISCHER STRUMPF**
MEDICAL SOCK
CHAUSSETTE MÉDICALE

(30) Priorität: 16.03.2016 DE 202016101471 U; 26.10.2016 DE 202016106016 U
(43) Veröffentlichungstag der Anmeldung: 23.01.2019
(73) Patentinhaber: FXF GmbH, 92224 Amberg (DE)
(72) Erfinder: FISCHER, Franz, 92224 Amberg (DE)
(74) Vertreter: Benninger, Johannes
(86) Internationale Anmeldenummer: PCT/EP2017/055856
(87) Internationale Veröffentlichungsnummer: WO 2017/157857

(56) Entgegenhaltungen:
- EP-B1- 2 120 810
- DE-U1-202015 007 262
- JP-A- 2010 124 926
- JP-A- 2012 000 168
- US-A- 3 119 390
- US-A1- 2011 088 145

## Beschreibung

Die vorliegende Erfindung betrifft einen medizinischen Strumpf gemäß den Merkmalen des Oberbegriffes des Anspruches 1.

Fußfehlstellungen, insbesondere Hallux Valgus, treten krankheits- und/oder altersbedingt und/oder durch eine falsche Fußbekleidung in der Bevölkerung regelmäßig auf. Bei Hallux Valgus verändert die Großzehe ihre Position und wandert, ausgelöst durch eine Abflachung der inneren Längswölbung am Fuß und eine Außenrotation im Unterschenkel, nach außen ab. Ein Hallux Valgus führt bei den Betroffenen zu Gehbinderungen und Schmerzen.

Hallux Valgus kann mittels Röntgenbild bildgebend und/oder mittels des Hallux Valguswinkels klinisch in verschiedene Schweregrade eingeteilt werden. Je nach Schweregrad stehen zur Therapie des Hallux Valgus konservative Verfahren, beispielsweise im Rahmen der Physiotherapie sowie der Orthopädietechnik, und/oder operative Verfahren zur Verfügung, wobei die Auswahl des Verfahrens zur Korrektur der Fehlstellung vom Schweregrad der Fehlstellung des Betroffenen abhängig ist. Zu den konservativen Verfahren gehören beispielsweise Schienen, Bandagen/Tapes und Silikonkeile.

Die Anwendung einer Schiene zur Korrektur von Fußfehlstellungen, insbesondere von Hallux Valgus, ist beispielsweise durch die DE 8 902 545 U1 offenbart. Bei der Schiene handelt es sich um einen Plastik- und/oder Metallstab, welcher durchgehend und medial von der Großzehe bis zur Ferse verläuft. Nachteilig ist, dass die Schiene sehr viel Platz einnimmt und das Tragen im Schuh fast unmöglich macht bzw. es zu Bewegungseinschränkungen des Betroffenen führt. Bei einem längeren Tragen führt die Schiene zu Druck- und Reibstellen am Fuß, so dass mit diesem Gegenstand eine Behandlung von längerer Dauer ausgeschlossen ist.

Silikonkeile, wie beispielsweise durch die DE 20 2010 013 804 U1 offenbart, werden zur Korrektur von Fuß- und/oder Beinfehlstellungen, insbesondere zur Korrektur von Hallux Valgus, zwischen der Großzehe und der zweiten Zehe positioniert. Die Anwendung von Silikonkeilen bringt die Nachteile mit sich, dass das Grundgelenk der zweiten Zehe stark belastet wird und es zur Luxation der zweiten Zehe im Grundgelenk kommt.

Eine weitere häufige Fußerkrankung stellt die Hammerzehe dar. Bei der Hammerzehe handelt es sich um eine permanente krallenartige Beugung der Zehe. Es kann zwischen einer flexiblen und einer fixierten Hammerzehe unterschieden werden. Wie bereits beim Krankheitsbild des Halux Valgus kann die Hammerzehe krankheits- und/oder altersbedingt und/oder durch eine falsche Fußbekleidung auftreten.

Durch die GB 445 921 A ist ein Mittel zur Korrektur von Fußfehlstellungen, insbesondere von Zehengelenken, wie beispielsweise bei einer Hammerzehe, offenbart. Auf einer Plattform kann eine Schlaufe vorgesehen sein, in welche Schlaufe die fehlgebildete Zehe eingeführt werden kann. Die Größe der Schlaufe und damit die auf den Zeh wirkende Kraft kann mittels einer Schnalle eingestellt werden.

Eine weitere Lösung zur Behandlung von Hammerzehen ist durch GB 267 325 A offenbart. Auf einer starren oder halbstarren Plattform können im Zehenbereich Schlitze vorgesehen sein, in welche Schlitze ein Band einfädelbar ist. Durch das Einfädeln des Bandes können Schlaufen für die Zehen bereitgestellt werden. Die auf die Zehen wirkenden Kräfte können über ein Anziehen und/oder Lösen des Bandes eingestellt werden.

Im Stand der Technik ist jedoch nachteilig, dass derartige Mittel zur Korrektur von Fußfehlstellungen für den Betroffenen, insbesondere beim Tragen im Schuh, sehr unangenehm ist. Zudem kann es aufgrund der Schlaufe auch zu Druckstellen an den Füßen und/oder den Zehen kommen.

Um Druckstellen an den Füßen zu vermeiden, ist im Stand der Technik bekannt, sog. Tapes zu verwenden. Mittels Tapes können zwar ebenfalls Fußfehlstellungen, wie beispielsweise Hallux Valux Valgus oder Hammerzehen, behandelt werden, jedoch kann es bei einem längeren Tragen von Tapes es zu Hautirritationen kommen.

Bei operativen Eingriffen wird durch eine Umstellungsosteotomie im Knochenbereich versucht, die Fehlstellung des Hallux Valgus und/oder der Hammerzehe zu korrigieren. Aufgrund des operativen Eingriffs werden der Band- und Kapselapparat des operierten Fußes vorübergehend geschwächt.

Um Fußerkrankungen, wie beispielsweise Hallux Valgus und/oder die Hammerzehe zu behandeln, sowie um das Operationsergebnis und den Bänderapparat nach einem operativen Eingriff zu stabilisieren, sind im Stand der Technik sogenannte medizinische Strümpfe bekannt.

Durch EP 2 120 810 B1 ist ein medizinischer Strumpf offenbart, bei welchem Zugbänder aus synthetischen und/oder natürlichen Fasern direkt in das Textil des Strumpfes eingearbeitet sind. Diese Fasern sind ganz oder teilweise um einen Rumpf des Fußes geführt sowie seitlich angeordnet und beaufschlagen eine erste Kammer des medizinischen Strumpfes für die Großzehe gegenüber einer zweiten Kammer für die weiteren Zehen mit einer Zugkraft. Die Fasern können auch vollständig um die Großzehe geführt werden.

Ein weiterer medizinischer Strumpf ist durch DE 20 2015 007 262 U1 vorbekannt, wonach ein elastisches Zugband auf das Textil des Strumpfes aufgebracht ist. Mittels des beschriebenen Strumpfes soll eine Schiefstellung bzw. ein Hallux Valgus der Großzehe korrigiert werden können, indem eine für die Großzehe vorgesehene erste Kammer mit einer Zugkraft gegenüber einer für die weiteren Zehen bereitgestellte zweite Kammer beaufschlagt wird. Wahlweise kann auch die zweite Kammer gegenüber der ersten Kammer mit einer Zugkraft beaufschlagt werden.

Bei den in der EP 2 120 810 B1 und DE 20 2015 007 262 U1 offenbarten medizinischen Strümpfen haben sich die Anordnung und Ausbildung der Zugbänder innerhalb des Textils des Strumpfes als nachteilig erwiesen. So besteht jeweils eine erhöhte Gefahr hinsichtlich einer Strangulation des Fußes, insbesondere des Rumpfbereichs sowie einzelner Zehen des Fußes.

Die US 2011/0088145 A1 beschreibt einen Strumpf zur Prophylaxe und/oder Behandlung von Fußverformungen. Der Strumpf umfasst an seiner medialen und lateralen Innenseite rutschsichere Mittel. Die rutschsicheren Mittel dienen dazu, den Strumpf an einer definierten Position am Fuß zu halten und ein Verrutschen des Strumpfes zu verhindern.

Die JP 2010 124926 A beschreibt ein Tape, welches zur Behandlung von Gelenken und/oder Muskeln unmittelbar auf die Haut geklebt wird. Das Tape umfasst an seinem Außenumfang Einkerbungen sowie mittig angeordnete Ausnehmungen. Durch die mittig angeordneten Ausnehmungen wird eine Schnittlinie definiert, so dass das Tape in zwei gleichbreite Streifen geschnitten werden kann.

Die JP 2012 000168 A beschreibt eine Bandage zu Korrektur von Fußfehlstellungen wie beispielsweise Hallux Valgus mit einem gestrickten mittleren Abschnitt und zwei elastischen äußeren Abschnitten. Der mittlere Abschnitt umfasst einen Schlitz, welcher dazu dient, den Halt bzw. die Position der Bandage am Fuß zu gewährleisten.

Die US 3 119 390 A beschreibt ein elastisches Polster, welches auf die Haut, insbesondere am Ballen, Zeh oder dergleichen aufgeklebt werden kann. Das Polster dient insbesondere dazu, Druck, Reibungen etc. von der verletzen oder schmerzenden Stelle fernzuhalten.

Der Erfindung liegt daher die Aufgabe zugrunde, einen medizinischen Strumpf zur Verfügung zu stellen, bei welchem die im Stand der Technik bekannten Nachteile zumindest teilweise überwunden werden können. Der medizinische Strumpf kann zugleich für eine prophylaktische Versorgung von Fuß- und/oder Beinfehlstellungen, insbesondere bei Hallux Valgus und/oder bei Hammerzehen, sowie für eine postoperative Therapie zur Sicherung des Operationsergebnisses dienen. Weiter können mit dem medizinischen Strumpf der Fuß, insbesondere dessen Bänder und Gelenke, nach einem operativen Eingriff stabilisiert werden. Darüber hinaus soll ein medizinischer Strumpf geschaffen werden, welcher an die Fußform und/oder an mögliche Schwellungen des Fußes anpassbar ist, ohne dabei den Fuß und/oder einzelne Zehen zu strangulieren.

Diese Aufgaben werden gelöst durch einen medizinischen Strumpf mit den Merkmalen im unabhängigen Anspruch 1. Weitere vorteilhafte Ausgestaltungen werden durch die Unteransprüche beschrieben.

Zur Lösung der Aufgabe schlägt die Erfindung einen medizinischen Strumpf mit den Merkmalen des unabhängigen Anspruchs 1 vor. Der medizinische Strumpf umfasst wenigstens zwei getrennte Kammern, wobei eine erste der wenigstens zwei getrennten Kammern zur Aufnahme einer Großzehe und wenigstens eine zweite Kammer zur Aufnahme mindestens einer weiteren Zehe ausgebildet sind. Der medizinische Strumpf umfasst mindestens ein elastisches Zugmittel zur Erzeugung einer definierten Zugkraft, welches mindestens eine elastische Zugmittel wenigstens ein elastisches Zugband umfasst, welches sich zumindest abschnittsweise entlang eines in die wenigstens zwei Kammern mündenden Rumpfbereich des Strumpfes erstreckt, wobei das wenigstens eine elastische Zugband an mindestens einer bestimmten Position wenigstens eine Ausnehmung besitzt.

Je nach Fuß- und/oder Beinfehlstellung kann der medizinische Strumpf mehrere Kammern für die Zehen bereitstellen. Es kann vorgesehen sein, dass der medizinische Strumpf wenigstens drei getrennte Kammern oder wenigstens vier getrennte Kammern oder fünf getrennte Kammern aufweist. Bei wenigstens drei getrennten Kammern kann die Großzehe von der ersten Kammer, die zweite Zehe von der zweiten Kammer und die weiteren Zehen von der dritten Kammer aufgenommen werden, wobei die dritte Kammer größer als die erste und zweite Kammer ausgebildet ist. Bei wenigstens vier getrennten Kammern kann die Großzehe von der ersten Kammer, die zweite Zehe von der zweiten Kammer, die dritte Zehe von der dritten Kammer und die weiteren Zehen von der vierten Kammer aufgenommen werden, wobei die vierte Kammer größer als die erste, zweite und dritte Kammer ausgebildet ist. Bei wenigstens fünf getrennten Kammern kann die Großzehe von der ersten Kammer, die zweite Zehe von der zweiten Kammer, die dritte Zehe von der dritten Kammer, die vierte Zehe von der vierten Kammer und die fünfte Zehe von der fünften Kammer aufgenommen werden. Damit kann der medizinische Strumpf für jeden Zeh eine separate Kammer bereitstellen.

Die definierte Zugkraft kann so gewählt sein, dass beim Tragen des medizinischen Strumpfes Fuß- und/oder Beinfehlstellung korrigiert werden. Bei Hallux Valgus kann insbesondere die Großzehe im Grundgelenk korrigiert werden, so dass die Großzehe über die Zeit ihre natürliche Position einnehmen kann bzw. dass die Großzehe nach einem operativen Eingriff die Soll-Beschaffenheit beibehalten kann. Die Zugkraft des wenigstens einen elastischen Zugbandes kann so ausgebildet sein, dass das Grundgelenk der mindestens einen weiteren Zehe, insbesondere der zweiten Zehe, nicht belastet und/oder deformiert wird. Durch eine gezielte Anordnung des mindestens einen Zugmittels auf einem Textil des Strumpfes kann die Zugkraft in Abhängigkeit des Krankheitsbildes genauestens dosiert werden. Zugleich können durch das Tragen des medizinischen Strumpfes im Fuß verlaufende Bänder und Gelenke stabilisiert werden. Eine Stabilisierung der im Fuß verlaufenden Bänder sowie der Gelenke kann vor allem nach einem operativen Eingriff zur Korrektur der Fuß- und/oder Beinfehlstellung, insbesondere eines Hallux Valgus, erforderlich sein. Durch Tragen des medizinischen Strumpfes kann der gesamte Fuß unterstützt und entlastet werden, wodurch Schmerzen gelindert werden.

Gemäß einer Ausführungsform kann sich das wenigstens eine elastische Zugmittel medial zumindest abschnittsweise entlang eines in die wenigstens zwei Kammern mündenden Rumpfbereichs des Strumpfes erstrecken, wobei die erste Kammer gegenüber der zweiten Kammer mit einer definierten Zugkraft beaufschlagt wird. Bei einer medialen Anordnung kann sich das wenigstens eine elastische Zugband von den wenigstens zwei Kammern und damit vom Vorderfuß, entlang des Mittelfußes bis zur Ferse erstrecken. Dadurch kann beispielsweise der Hallux Valguswinkel korrigiert werden, in dem die erste der wenigstens zwei getrennten Kammern gegenüber der wenigstens einen zweiten Kammer mit einer Zugkraft beaufschlagt wird.

Gemäß einer Ausführungsform des medizinischen Strumpfes kann sich das wenigstens eine elastische Zugband lateral zumindest abschnittsweise entlang eines in die wenigstens zwei Kammern mündenden Rumpfbereichs des Strumpfes erstrecken, wobei die zweite Kammer gegenüber der ersten Kammer mit einer definierten Zugkraft beaufschlagt wird. Bei einer lateralen Anordnung kann sich das wenigstens eine elastische Zugband von den wenigstens zwei Kammern, damit vom Vorderfuß und/oder vom Außenballen entlang zur Ferse erstrecken.

Erfindungsgemäß erfolgt eine Kombination, wonach sich das wenigstens eine elastische Zugband lateral und medial zumindest abschnittsweise entlang eines in die wenigstens zwei Kammern mündenden Rumpfbereichs des Strumpfes erstreckt. Das wenigstens eine elastische Zugband kann sich medial vom Bereich von den wenigstens zwei Kammern und damit vom Vorderfuß entlang des Mittelfußes bis zur Ferse erstrecken, während das lateral angeordnete wenigstens eine elastische Zugband als Gegenlager für das medial angeordnete wenigstens eine elastische Zugband dienen kann.

Das wenigstens eine elastische Zugband kann dafür sorgen, dass bei einem Tragen des medizinischen Strumpfes unerwünschte Bewegungen des Fußes und/oder der Zehen unterbunden werden. Die erforderliche Stärke bzw. Zugkraft des wenigstens einen elastischen Zugbandes zur Korrektur der Fuß- und/oder Beinfehlstellung, insbesondere zur Korrektur des Großzehs bei Hallux Valgus, kann vom Schweregrad der Erkrankung abhängen. Danach kann das wenigstens eine elastische Zugband je nach erforderlicher Zugkraft aus einer Schicht oder aus mehreren Schichten bestehen. Über die Anzahl an Schichten des wenigstens einen elastischen Zugbandes kann dessen Stärke bzw. Zugkraft gebildet werden. Bei wenigstens einem elastischen Zugband, welches aus wenigen Schichten, beispielsweise aus einer Schicht gebildet ist, kann eine verhältnismäßig geringere Zugkraft auf die erste Kammer gegenüber der wenigstens einen zweiten Kammer beaufschlagt werden, als bei wenigstens einem elastischen Zugband, welches aus einer Vielzahl von Schichten gebildet ist. Neben der Anzahl an Schichten kann die Zugkraft des wenigstens einen elastischen Zugbandes von der Wahl des Materials abhängen.

Die jeweils an mindestens einer bestimmten Position des wenigstens einen elastischen Zugbandes vorgesehenen wenigstens eine Ausnehmung kann unterschiedlich ausgebildet sein. Die wenigstens eine Ausnehmung kann für eine Verteilung der definierten Zugkraft sorgen, ohne dabei die Funktion des medizinischen Strumpfes zu beeinträchtigen. Wahlweise kann durch die wenigstens eine Ausnehmung die durch das wenigstens eine elastische Zugmittel erzeugte Zugkraft definiert bzw. zu dosiert werden, da die wenigstens eine Ausnehmung die auf den Strumpf wirkende Zugkraft beeinflusst.

Die wenigstens eine Ausnehmung kann sich zumindest abschnittsweise entlang der Längserstreckungsrichtung des wenigstens einen elastischen Zugbandes erstrecken. Wahlweise kann die wenigstens eine Ausnehmung schräg orientiert entlang der Längserstreckungsrichtung des wenigstens einen elastischen Zugbandes ausgebildet sein. Vorzugsweise kann die wenigstens eine Ausnehmung länglich und/oder geschwungen und/oder eckig ausgebildet sein. Die Größe und die Breite der wenigstens einen Ausnehmung kann von der Fuß- und/oder Beinfehlstellung abhängig sein.

Es kann vorgesehen sein, dass durch die wenigstens eine Ausnehmung eine Faltenbildung des wenigstens einen elastischen Zugbandes beim Tragen des medizinischen Strumpfes verhindert wird, da der medizinische Strumpf mit dem elastischen Zugband besser an die Fußform und gegebenenfalls auch bei Schwellungen des Fußes nach einem operativen Eingriff anpassbar ist.

Es kann auch vorgesehen sein, dass sich das wenigstens eine elastische Zugband in Richtung der wenigstens zwei Kammern verjüngt.

Durch die verjüngende Ausbildung des wenigstens einen elastischen Zugbands kann die definierte Zugkraft über den Rumpfbereich des Strumpfes ebenfalls dosiert werden. Das bedeutet, dass das elastische Zugband an denjenigen Stellen breiter ausgebildet sein kann, an welchen jeweils mehr Zugkraft und Stabilisierung erforderlich sind bzw. an den denjenigen Stellen schmäler zulaufen kann, an welchen jeweils weniger Zugkraft und Stabilisierung erforderlich sind. Wahlweise kann das wenigstens eine elastische Zugband mit zumindest weitgehend gleichbleibender Breite ausgebildet sein.

Eine Weiterbildung sieht vor, dass das wenigstens eine elastische und sich längsgerichtet zumindest abschnittsweise entlang des in die wenigstens zwei Kammern mündenden Rumpfbereichs erstreckende Zugband die wenigstens zwei getrennten Kammern zumindest näherungsweise erreichen kann.

Die Länge des wenigstens einen elastischen Zugbandes kann je nach Fuß- und/oder Beinfehlstellung sowie Fußgröße variieren, um eine definierte Zugkraft zu erzeugen.

Erfindungsgemäß umfasst das mindestens eine elastische Zugmittel wenigstens ein weiteres elastisches Zugband, welches wenigstens eine weitere elastische Zugband zumindest abschnittsweise um den Rumpfbereich geführt ist.

Das zumindest abschnittsweise um den Rumpfbereich geführte wenigstens eine weitere elastische Zugband kann für eine Stabilisierung des Rumpfbereichs in diesem Abschnitt sorgen. Bei einem Tragen des medizinischen Strumpfs würde das wenigstens eine weitere elastische Zugband von der medialen Seite des Fußes dorsal über den Fußrücken nach proximal und distal zur Fußaußenseite des Mittelfußes verlaufen.

In einer weiteren Ausführungsform kann das wenigstens eine weitere Zugband auch so angeordnet sein, dass bei einem Tragen des medizinischen Strumpfes das wenigstens eine weitere elastische Zugband von der medialen Seite des Fußes plantar nach proximal und distal zur Fußaußenseite des Mittelfußes verläuft.

Eine zweckmäßige Weiterbildung der Erfindung sieht vor, dass das wenigstens eine elastische Zugband und das wenigstens eine weitere elastische Zugband schräg zueinander orientiert sein können.

Es kann auch vorgesehen sein, dass das wenigstens eine weitere elastische Zugband das mindestens eine Zugband zumindest näherungsweise erreicht oder bei welchem das wenigstens eine weitere elastische Zugband und das mindestens eine Zugband ineinander übergehen.

Je nach Ausgestaltung können das wenigstens eine weitere elastische Zugband und das wenigstens eine Zugband einteilig ausgebildet sein, d.h. das sie können ineinander übergehen. Alternativ können das wenigstens eine weitere elastische Zugband und das wenigstens eine Zugband mehrteilig, vorzugsweise zweiteilig, ausgebildet sein.

Erfinderungsgemäß besitzt das wenigstens eine weitere elastische Zugband an mindestens einer bestimmten Position wenigstens eine weitere Ausnehmung, welche wenigstens eine weitere Ausnehmung sich zumindest abschnittsweise entlang der Längserstreckungsrichtung des wenigstens einen weiteren elastischen Zugbandes erstreckt.

Die jeweils an mindestens einer bestimmten Position des wenigstens einen weiteren elastischen Zugbands vorgesehene wenigstens eine weitere Ausnehmung kann unterschiedlich ausgebildet sein. Die wenigstens eine weitere Ausnehmung kann für eine Verteilung der definierten Zugkraft sorgen, ohne dabei die Funktion des medizinischen Strumpfes zu beinträchtigen. Wahlweise kann die durch das wenigstens eine elastische Zugmittel erzeugte Zugkraft definiert bzw. dosiert werden, da über die wenigstens eine Ausnehmung die auf den Strumpf wirkende Zugkraft beinflussbar ist. Die Form und Anzahl der Ausnehmungen können je nach Ausführungsform des medizinischen Strumpfs variieren. So kann die Ausnehmung länglich und/oder geschwungen und/oder eckig ausgebildet sein.

Es kann vorgesehen sein, dass sich die wenigstens eine Ausnehmung des wenigstens einen weiteren elastischen Zugbands zumindest abschnittsweise über den Rumpfbereich des Strumpfes erstreckt. Die wenigstens eine weitere Ausnehmung kann als Schlitz ausgebildet sein. Wahlweise kann die wenigstens eine weitere Ausnehmung einem Strangulieren des Fußes, insbesondere des Rumpfbereichs beim Tragen des medizinischen Strumpfes entgegenwirken.

Gehen das wenigstens eine elastische Zugband und das wenigstens eine weitere elastische Zugband ineinander über, kann vorgesehen sein, dass sich die wenigstens eine Ausnehmung des wenigstens einen elastischen Zugbandes zumindest abschnittsweise über das wenigstens eine weitere elastische Zugband erstreckt. Die wenigstens eine Ausnehmung kann eine geschwungene Form ausbilden. Die geschwungene Form der wenigstens einen Ausnehmung kann dazu beitragen, dass der medizinische Strumpf mit dem mindestens einen elastischen Zugmittel besser an die Fußform, insbesondere bei Schwellungen nach einem operativen Eingriff anpassbar ist.

Es kann vorgesehen sein, dass das mindestens eine elastische Zugmittel wenigstens ein weiteres elastisches Zugband umfasst, welches sich zumindest abschnittsweise entlang einer in die wenigstens zwei Kammern mündender Sohle des Strumpfes erstreckt und zumindest eine Kammer der wenigstens zwei getrennten Kammern zumindest abschnittsweise umgreift.

Das wenigstens eine elastische Zugband kann an einer Unterseite des Strumpfes angeordnet sein. Das wenigstens eine elastische Zugband kann sich von Ferse bis zum Fußballen des Strumpfes erstrecken, insbesondere bis zu zumindest einer der wenigstens zwei getrennten Kammern erstrecken.

Das wenigstens eine weitere elastische Zugband kann eine definierte Zugkraft ausbilden, welche vorzugsweise vertikal nach unten gerichtet ist. Die definierte Zugkraft kann über die Schichtanzahl des wenigstens einen elastischen Bandes eingestellt werden, wobei die Zugkraft umso größer ist, aus je mehr Schichten das wenigstens eine elastische Zugband gebildet ist. Umgekehrt kann gelten, dass die Zugkraft umso geringer ausfällt, je weniger Schichten das wenigstens eine elastische Band umfasst. Die Stärke der vertikal nach unten gerichteten Zugkraft kann von der Schichtanzahl abhängig sein.

Aufgrund die durch das wenigstens eine weitere elastische Zugband vertikal erzeugte Zugkraft nach unten kann die in der zumindest einen Kammer befindliche Zehe korrigiert werden, wobei insbesondere Fußfehlstellung, wie beispielsweise Hammerzehen, korrigiert werden können.

Es kann vorgesehen sein, dass sich das wenigstens eine weitere elastische Zugband im Bereich von zumindest einer der wenigstens zwei getrennten Kammern in zumindest zwei elastische Zugbandstreifen aufteilt. Die zumindest zwei elastischen Zugbandstreifen können zumindest näherungsweise gleichbreit ausgebildet sein. Vorzugsweise kann sich das wenigstens eine elastische Zugband insbesondere im Bereich der zweiten Kammer in zumindest näherungsweise zwei gleichbreite Zugbandstreifen aufteilen. Das wenigstens eine elastische Zugband kann in diesem Abschnitt gabelförmig ausgebildet sein, so dass das wenigstens eine elastische Zugband um wenigstens eine der wenigstens zwei Kammern führbar ist.

Das wenigstens eine weitere elastische Zugband, insbesondere die zumindest zwei elastischen Zugbandstreifen, kann/können um zumindest eine der wenigstens zwei getrennten Kammern geführt werden. Es kann vorgesehen sein, dass sich Enden der zumindest zwei elastischen Zugbandstreifen zumindest näherungsweise erreichen oder überlappen.

Das wenigstens eine elastische Zugband kann zumindest eine der wenigstens zwei getrennten Kammern vollständig umgreifen, wobei jeweils die Enden des wenigstens einen elastischen Zugbandes überlappen. Das wenigstens eine elastische Band kann mehrfach und/oder einfach um wenigstens eine der wenigstens zwei getrennten Kammern geführt werden.

Alternativ kann jeder Kammer mindestens ein elastisches Zugband zugeordnet sein, um Fußfehlstellungen mit einer vertikal nach unten gerichteten Zugkraft korrigieren zu können. So kann beispielsweise der ersten Kammer und/oder der zweiten Kammer und/oder der dritten Kammer und/oder der vierten Kammer und/oder der fünften Kammer jeweils wenigstens ein elastisches Zugband zugeordnet sein.

Es kann vorgesehen sein, dass die Stärke der definierten Zugkraft über die Form und/oder über die Breite des wenigstens einen weiteren elastischen Zugbandes einstellbar ist. Je breiter das wenigstens eine weitere elastische Zugband ausgebildet ist, desto mehr kann eine vertikal nach unten gerichtete Zugkraft aufgebracht werden. Je schmäler das wenigstens eine elastische Zugband ausgebildet ist, desto weniger kann eine vertikal nach unten gerichtete Zugkraft aufgebracht werden. Wahlweise kann das wenigstens eine weitere Zugband an mindestens einer bestimmten Position wenigstens eine weitere Ausnehmung umfassen, welche sich vorzugsweise entlang der Längserstreckungsrichtung des wenigstens einen weiteren elastischen Zugbandes erstreckt, um die Zugkraft einzustellen.

Es kann vorgesehen sein, dass sich das wenigstens eine weitere elastische Zugband in Richtung der Sohle und/oder der Ferse des Strumpfs verjüngt. Eine verjüngende Ausbildung des wenigstens einen elastischen Zugbandes kann dazu beitragen, die auf die zweite Kammer beaufschlagte und vertikal nach unten gerichtete Zugkraft zu dosieren, wobei die Zugkraft in Richtung des verjüngenden Endes des wenigstens einen elastischen Bandes abnimmt.

Gemäß einer Weiterbildung der Erfindung kann die Länge des wenigstens einen elastischen Zugbandes unterschiedlich gewählt sein. Insbesondere kann die Länge des wenigstens einen elastischen Bandes an die jeweilige Fußform individuell angepasst sein.

Gemäß einer Weiterbildung der Erfindung kann vorgesehen sein, dass die definierte Zugkraft über die wenigstens eine Ausnehmung und/oder über die wenigstens eine weitere Ausnehmung einstellbar und/oder dosierbar ist.

Es kann auch vorgesehen sein, dass der medizinische Strumpf zumindest bereichsweise durch Textil gebildet ist, wobei das mindestens eine elastische Zugmittel auf das Textil aufgebracht oder in das Textil eingearbeitet ist. Das mindestens eine elastische Zugmittel kann auf das Textil des Strumpfes laminiert und/oder gegossen und/oder in das Textil eingewebt und/oder eingestrickt oder dergleichen werden.

Das mindestens eine elastische Zugmittel und/oder das wenigstens eine weitere elastische Zugband können auf der Außenseite des Strumpfes aufgebracht werden, wodurch die Haut geschont wird und Hautirritationen vermieden werden.

Es kann vorgesehen sein, dass das mindestens eine elastische Zugband durch einen Thermoplast gebildet sind. Es können auch weitere elastische Materialien verwendet werden.

Weiterhin wird ein Verfahren zur Herstellung eines medizinischen Strumpfes beschrieben, welches folgende Schritte aufweist:
In einem ersten Schritt werden bestimmte Charakteristika des Fußes festgestellt.
Es können Fuß- und/oder Beinfehlstellungen, wie beispielsweise Hallux Valgus oder Hammerzehen visuell erkannt und beurteilt werden. Bei beispielsweise einem Hallux Valgus kann der Grad der Verformung über den Hallux Valguswinkel festgestellt werden. Alternativ kann die Fehlstellung beispielsweise mittels Röntgendiagnostik dargestellt werden.

In einem weiteren Schritt wird die Soll-Beschaffenheit mindestens eines elastischen Zugmittels unter Berücksichtigung der festgestellten Charakteristika ermittelt. Das mindestens eine elastische Zugmittel kann wenigstens ein elastisches Zugband und/oder wenigstens ein weiteres elastisches Zugband umfassen, welches wenigstens eine elastische Zugband und/oder wenigstens ein weiteres elastisches Zugband als thermoplastisches Band ausgebildet sind.

Bei der Ermittlung der Soll-Beschaffenheit des mindestens einen elastischen Zugmittels kann definiert werden, aus wie vielen Schichten das wenigstens eine elastische Zugband und/oder das wenigstens eine weitere elastische Zugband gebildet werden soll. Über die Anzahl an Schichten kann die Stärke bzw. die Zugkraft des wenigstens einen elastischen Zugbandes und/oder des wenigstens einen weiteren elastischen Zugbandes zur Korrektur von Fuß- und/oder Beinfehlstellung definiert werden. Es kann auch definiert werden, an welcher Position das mindestens eine elastische Zugmittel wenigstens eine Ausnehmung und/oder wenigstens eine weitere Ausnehmung besitzen soll, um die Kräfte zu dosieren bzw. einzustellen.

In einem nächsten Schritt des Verfahrens wird das mindestens eine elastische Zugmittel mit der Soll-Beschaffenheit auf ein Textil des Strumpfes aufgebracht oder in das Textil des Strumpfes eingearbeitet, woraus resultierend ein medizinischer Strumpf aus dem Strumpf zusammen mit dem elastischen Zugmittel wird.

Das Aufbringen des mindestens einen elastischen Zugmittels kann unter Temperatureinwirkung erfolgen. Zugleich kann das mindestens eine elastische Zugmittel und/oder das mindestens eine Beaufschlagungsmittel auf das Textil des Strumpfes laminiert und/oder gegossen werden.

Es kann auch vorgesehen sein, dass wenigstens zwei Kammern gebildet werden, wobei eine erste der wenigstens zwei getrennten Kammern zur Aufnahme einer Großzehe und wenigstens eine zweite Kammer zur Aufnahme mindestens einer weiteren Zehe ausgebildet sind. Es können auch wenigstens drei getrennte Kammern oder wenigstens vier getrennte Kammern oder fünf getrennte Kammern gebildet werden.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

Fig. 1 zeigt eine schematische Perspektivansicht des medizinischen Strumpfes.

Die Figuren 2A bis 2E zeigen jeweils eine weitere Ausführungsform des medizinischen Strumpfes in verschiedenen Ansichten.

Die Figuren 3A und 3B zeigen jeweils ein weiteres Ausführungsbeispiel des medizinischen Strumpfes in verschiedenen Ansichten.

Die Figuren 4A bis 4D zeigen jeweils schematische Ansichten des medizinischen Strumpfes in verschiedenen Ansichten.

Die Fig. 5 zeigt eine weitere Ausführungsform des medizinischen Strumpfes gemäß der Figuren 4A bis 4D in einer Ansicht von unten.

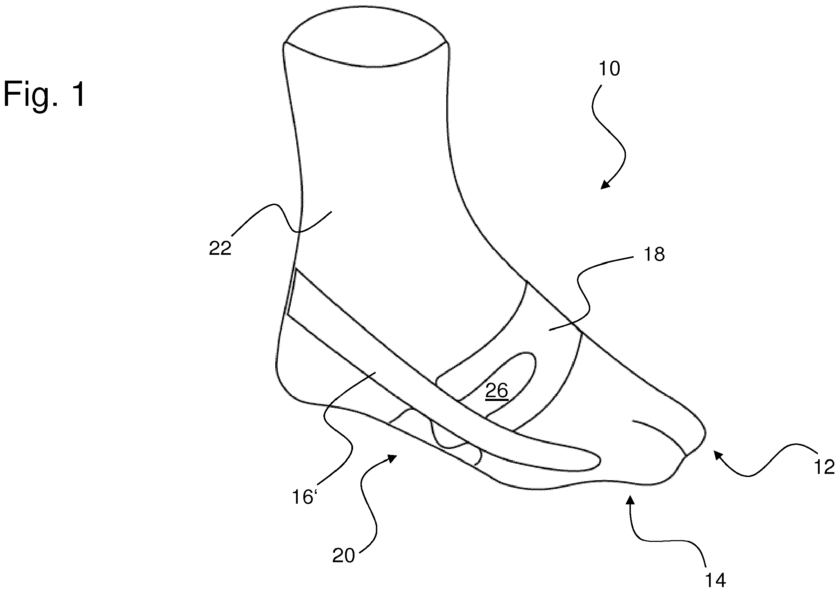

Für gleiche oder gleich wirkende Elemente der Erfindung werden in den Figuren 1 bis 5 jeweils identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung ausgestaltet sein kann und stellen keine abschließende Begrenzung dar.

Die schematische Perspektivansicht der Fig. 1 zeigt einen medizinische Strumpf 10, welcher typischerweise zur Korrektur von Fuß- und/oder Beinfehlstellungen, insbesondere von Hallux Valgus, eingesetzt wird. Der medizinische Strumpf 10 umfasst wenigstens zwei getrennte Kammern 12 und 14. Die wenigstens zwei getrennte Kammern 12 und 14 setzen sich aus wenigstens einer ersten Kammer 12, in welche die Großzehe aufgenommen werden kann, sowie aus wenigstens einer zweiten Kammer 14, in welche die weiteren Zehen aufgenommen werden können, zusammen. Der medizinische Strumpf 10 weist mindestens ein elastisches Zugmittel, hier in Form wenigstens eines elastischen Zugbandes 16 auf. Das wenigstens eine elastische Zugband 16 erstreckt sich medial (hier nicht dargestellt) längsgerichtet zumindest abschnittsweise entlang eines in die wenigstens zwei Kammern mündenden Rumpfbereichs 20 des Strumpfes 10. Das medial angeordnete wenigstens eine elastische Zugband 16 läuft vom Bereich der Ferse bis zur Großzehe, so dass das wenigstens eine elastische Zugband 16 zur Beaufschlagung der ersten Kammer 12 gegenüber der wenigstens einen zweiten Kammer mit einer Zugkraft ausgebildet ist. Die Zugkraft sorgt dafür, dass die Großzehe leicht von den übrigen Zehen weggezogen und damit zumindest näherungsweise in seine natürliche Position gebracht wird. In der Fig. 1 ist lediglich das lateral angeordnete wenigstens eine elastische Zugband 16' sichtbar, welches sich vom Bereich der Ferse bis zum Außenballen erstreckt. Das lateral angeordnete wenigstens eine elastische Zugband 16' dient als Gegenlager gegenüber dem medial angeordneten wenigstens einem elastischen Zugband 16.

Die Zugkraft des - hier in Fig. 1 nicht sichtbaren - wenigstens einen elastischen Zugbands 16 ist so gewählt, dass bei einer Behandlung von Hallux Valgus die Großzehe im Grundgelenk korrigiert wird und über die Zeit, d.h. nach längerem Tragen des medizinischen Strumpfes 10, ihre natürliche Position einnimmt. Der medizinische Strumpf 10 dient einerseits kosmetischen Zwecken, um störende Fußfehlstellungen und Zehenstellungen zu korrigieren, kann jedoch darüber hinaus insbesondere auch zur postoperativen Therapie eingesetzt werden, um Operationsergebnisse zu sichern und zu verhindern, dass der korrigierte Zeh wieder in eine Fehlstellung zurückfällt. Zugleich sorgt der medizinische Strumpf 10 für eine Stabilisierung der im Fuß verlaufenden Bänder und Gelenke, indem durch die elastischen Zugbänder 16 und 16' gezielt Kräfte in den Fuß eingeleitet werden.

Dem medizinischen Strumpf 10 ist wenigstens ein weiteres elastisches Zugband 18 zugeordnet, welches wenigstens eine weitere elastisches Zugband 18 zumindest abschnittsweise um den Rumpfbereich 20 geführt ist und damit in etwa quer zur Längserstreckungsrichtung der Zugbänder 16 bzw. 16' verläuft. Das wenigstens eine weitere elastische Zugband 18 erstreckt sich in der Fig. 1 in etwas quer zur Längsrichtung des Fußes über den Mittelfuß und sorgt bei einem Tragen des medizinischen Strumpfes 10 für eine Stabilisierung des Fußes und der im Fuß verlaufenden Bänder und Gelenke.

Das wenigstens eine elastische Zugband 16 und das wenigstens eine weitere elastische Zugband 18 sind jeweils mehrteilig ausgebildet, wodurch das wenigstens eine weitere elastische Zugband 18 das wenigstens eine elastische Zugband 16 zumindest näherungsweise erreicht. Das wenigstens eine elastische Zugband 16, 16' umfasst an mindestens einer bestimmten Position wenigstens eine Ausnehmung, welche in Fig. 1 nicht dargestellt ist. Das wenigstens eine weitere elastische Zugband 18 weist an mindestens einer bestimmten Position wenigstens eine weitere Ausnehmung 26 auf, so dass das wenigstens eine weitere elastische Zugband 18 eine gabelförmige Form ausbildet. Über die Länge und Breite der wenigstens einen weiteren Ausnehmung 26, die das weitere elastische Zugband 18 in dessen Längserstreckungsrichtung unterteilt, wird die auf den Rumpfbereich 20 wirkende bzw. den Rumpfbereich 20 beaufschlagende Zugkraft dosiert. Außerdem kann sich der medizinische Strumpf 10 besser an die Fußform, insbesondere auch bei Schwellungen nach einem operativen Eingriff, anpassen. Besonders hierfür ist sind die Ausnehmungen 26 wichtig, weil sie zu große und einen Heilungsverlauf beeinträchtigende Druckkräfte, die ansonsten von den Zugbändern 16, 16' und/oder 18 auf das darunter liegende Fußgewebe ausgeübt würden, spürbar reduziert und besser im Fußbereich verteilt.

Der medizinische Strumpf 10 ist zumindest bereichsweise durch Textil 22 gebildet. Das wenigstens eine elastische Zugband 16, 16' sowie das wenigstens eine weitere elastische Zugband 18 sind vorzugsweise aus einem thermoplastischen Kunststoffmaterial gebildet. Unter Temperatureinwirkung werden das wenigstens eine elastische Zugband 16, 16' und/oder das wenigstens eine weitere elastische Zugband 18 auf das Textil 22 aufgebracht, so dass der Strumpf 10 und das wenigstens eine elastische Zugband 16, 16' und das wenigstens eine weitere elastische Zugband 18 einen medizinischen Strumpf 10 ausbilden. Das wenigstens eine elastische Zugband 16 und/oder das wenigstens eine weitere elastische Zugband 18 befindet sich außen am Strumpf 10, ist jedoch durch die zuvor eingebrachte Temperatureinwirkung stoffschlüssig mit dem Textilmaterial 22 des Strumpfes 10 verbunden und kann dadurch wirksam Kräfte übertragen, die ansonsten über das Gestricke oder Gewebe des Textilmaterials 22 über den Fuß verteilt und damit unkontrolliert in alle Richtungen abgebaut würden.

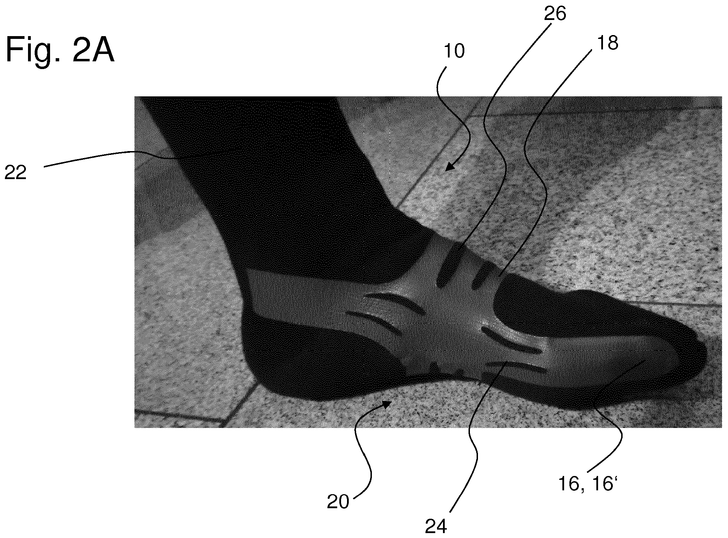

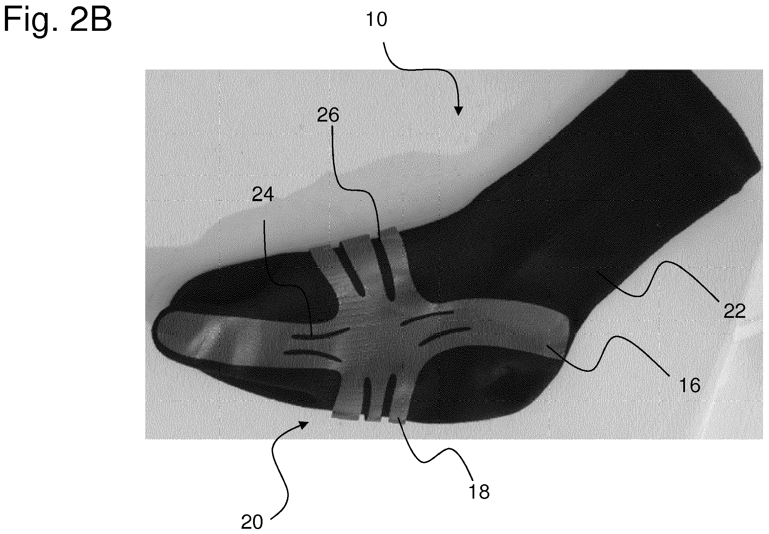

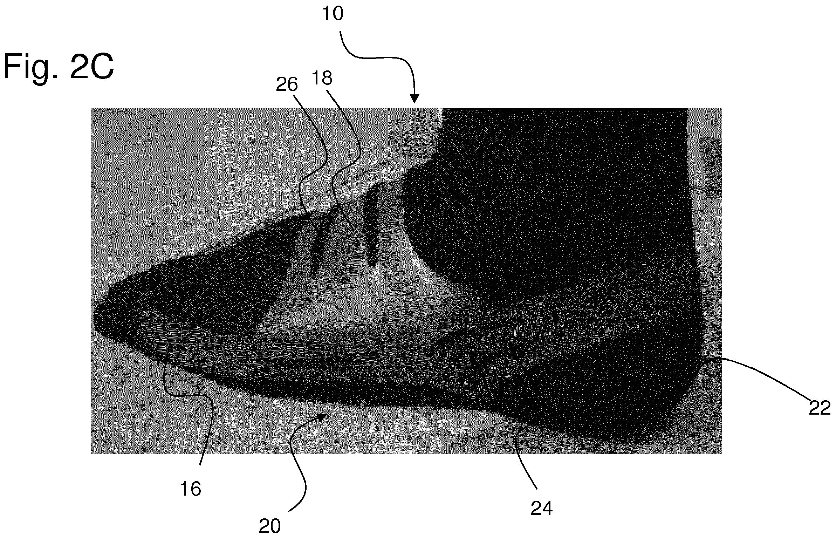

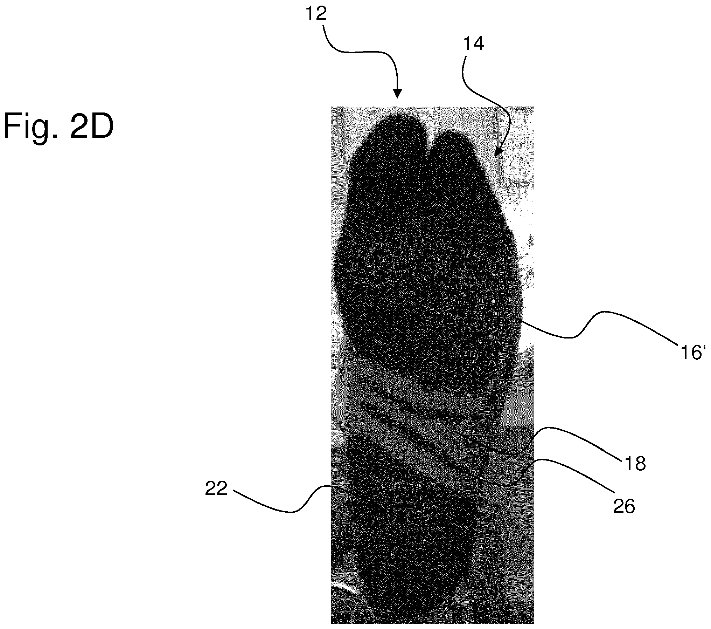

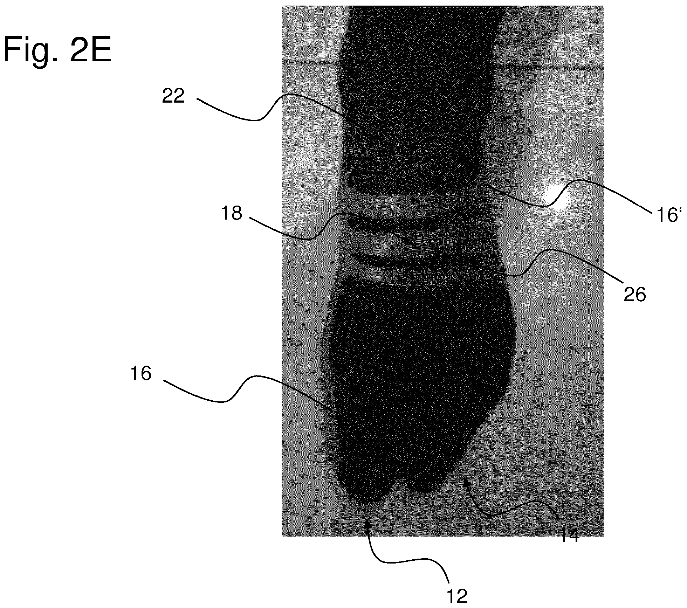

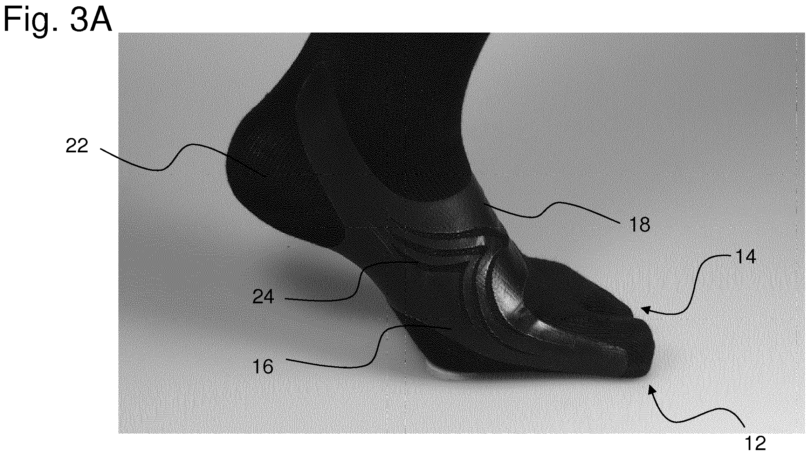

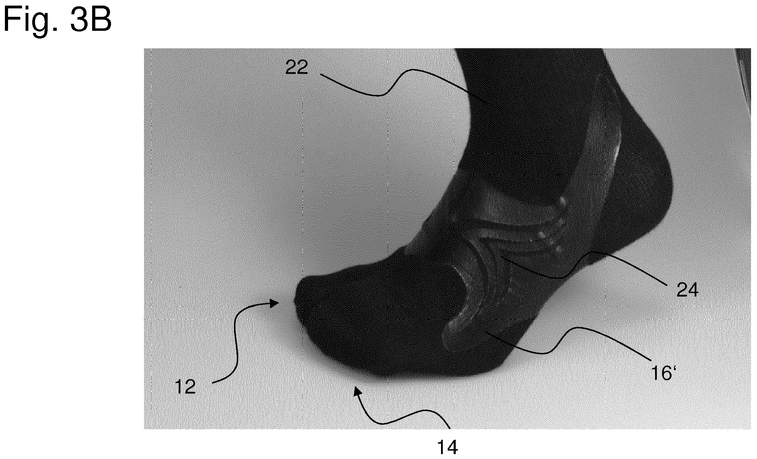

Die Figuren 2A bis 2E sowie die Figuren 3A und 3B zeigen jeweils weitere Ausführungsformen des medizinischen Strumpfes 10 in mehreren Ansichten. Das wenigstens eine elastische Zugband 16, 16' und das wenigstens eine weitere elastische Zugband 18 gehen hierbei ineinander über und sind insgesamt einteilig ausgebildet. Auch bei dieser Ausführungsform dient der medizinische Strumpf 10 neben kosmetischen Zwecken zur prophylaktischen Versorgung von Fuß- und/oder Beinfehlstellung, insbesondere bei Hallux Valgus, und/oder zur postoperativen Therapie, um das Operationsergebnis zu sichern.

Der medizinische Strumpf 10 weist jeweils wenigstens zwei getrennte Kammern auf. Eine erste der wenigstens zwei getrennten Kammern 12 ist zur Aufnahme einer Großzehe ausgebildet und eine zweite der wenigstens zwei getrennten Kammern 14 ist zur Aufnahme wenigstens einer weiteren Zehe, vorzugsweise der vier Kleinzehen, ausgebildet. Der medizinische Strumpf 10 umfasst mindestens ein elastisches Zugmittel zur Erzeugung einer definierten Zugkraft. Das mindestens eine elastische Zugmittel umfasst wenigstens ein medial angeordnetes elastisches Zugband 16 und wenigstens ein lateral angeordnetes elastisches Zugband 16'. Das wenigstens eine medial angeordnete elastische Zugband 16 ist zur Beaufschlagung der ersten Kammer 12 gegenüber der wenigstens einen zweiten Kammer 14 mit einer Zugkraft ausgebildet. Durch die beaufschlagte Zugkraft wird die Großzehe in ihrer Position korrigiert. Das wenigstens eine elastische Zugband 16, 16' ist aus einem thermoplastischen Material gebildet und umfasst mehrere Schichten. Über die Anzahl an Schichten des thermoplastischen Materials des wenigstens einen elastischen Zugbands 16, 16' und des wenigstens einen weiteren elastischen Zugbands 18 kann die Stärke bzw. die Zugkraft des wenigstens einen elastischen Zugbands 16, 16' und/oder des wenigstens einen weiteren elastischen Zugbands 18 gebildet werden.

Das wenigstens eine elastische Zugband 16, 16' erstreckt sich sowohl lateral als auch medial längsgerichtet zumindest abschnittsweise entlang eines in die wenigstens zwei Kammern mündenden Rumpfbereichs 20 des Strumpfes, wobei das wenigstens eine elastische Zugband 16, 16' bei der Ausführungsform der Figuren 2A bis 2E um den Rumpfbereich 20 geführt ist. Dabei erreicht das wenigstens eine elastische und sich längsgerichtet zumindest abschnittsweise entlang des in die wenigstens zwei Kammern mündenden Rumpfbereichs 20 erstreckende Zugband 16 die wenigstens zwei getrennten Kammern zumindest näherungsweise.

Das medial angeordnete wenigstens eine elastische Zugband 16 erstreckt sich vom Bereich der Ferse bis zum Bereich des Vorderfußes, insbesondere bis zur Großzehe. Das medial angeordnete wenigstens eine elastische Zugband 16 dient zur Korrektur der Großzehe, da durch das wenigstens eine elastische Zugband 16 die wenigstens eine erste Kammer 12 gegenüber der wenigstens einen zweiten Kammer 14 mit Zugkraft beaufschlagt wird.

Das lateral angeordnete wenigstens eine elastische Zugband 16'erstreckt sich vom Bereich der Ferse bis zum Außenballen und dient als Gegenlager zum medial angeordneten wenigstens einen elastischen Zugband 16.

Neben dem wenigstens einem elastischen Zugband 16 ist wenigstens ein weiteres elastisches Zugband 18 vorgesehen, welches wenigstens eine weitere elastische Zugband 18 zumindest abschnittsweise um den Rumpfbereich 20 geführt ist. Das wenigstens eine weitere elastische Zugband 18 befindet sich im Bereich des Mittelfußes und sorgt in diesem Bereich beim Tragen des medizinischen Strumpfes 10 für eine Stabilisierung des Fußes und der im Fuß verlaufenden Bänder und Gelenke.

Gemäß der Figuren 2A bis 2E umfasst das wenigstens eine elastische Zugband 16, 16' an mindestens einer bestimmten Position wenigstens zwei Ausnehmungen 24. Die wenigstens zwei Ausnehmungen 24 umfassen jeweils ein Paar von Schlitzen, welche sich jeweils entlang der Längserstreckungsrichtung des wenigstens einen elastischen Zugbandes 16, 16' erstrecken. Die schlitzartigen Ausnehmungen 24 können bspw. jeweils eine Länge aufweisen, die zwischen 10% und ca. 30% der Gesamtlänge des medialen und/oder lateralen elastischen Zugbandes 16, 16' entsprechen kann. Die Länge der schlitzartigen Ausnehmung 24 kann jeweils variieren, so dass die verschiedenen Ausnehmungen 24 jeweils unterschiedlich lang sein können. Die Ausnehmungen 24 können zudem leicht gekrümmt sein, wie dies die Figuren 2A bis 2E andeutungsweise erkennen lassen, verlaufen mit ihrer Hauptrichtung jedoch in etwa parallel zur Längserstreckungsrichtung der jeweiligen Zugbänder 16, 16'. Außerdem kann die Breite der jeweiligen Ausnehmung 24 in etwa zwischen 10% und 50% der Breite des jeweiligen Zugbandes 16, 16' an der Stelle der Ausnehmung 24 betragen. Jedes Zugband 16, 16' kann wahlweise nur eine Ausnehmung 24 über seiner Breite aufweisen, während mehrere separate und voneinander beabstandete Ausnehmungen 24 über den Längenverlauf der Zugbänder 16, 16' vorhanden sein können. Es können sich gemäß Figuren 2A bis 2E auch jeweils paarweise nebeneinander angeordnete Ausnehmungen 24 in den Zugbändern 16, 16' befinden, die über die Breite des jeweiligen Zugbandes 16, 16' so voneinander beabstandet sind, dass drei etwa gleichbreite Streifen an den Stellen der Ausnehmungen 24 gebildet sind.

Das wenigstens eine weitere elastische Zugband 18 besitzt an mindestens einer bestimmten Position wenigstens zwei weitere Ausnehmungen 26, welche sich entlang der Längserstreckungsrichtung des wenigstens einen weiteren elastischen Zugbandes 18 erstrecken. Die wenigstens zwei weiteren Ausnehmungen 26 sind zueinander beabstandet sowie auf einer Ober- und Unterseite des Rumpfbereichs 20 des Strumpfes 10 angeordnet. Auch hier gilt, dass die schlitzartigen weiteren Ausnehmungen 26 bspw. jeweils eine Länge aufweisen können, die zwischen 10% und ca. 80% der Gesamtbreite des Fußes bzw. der Länge des weiteren elastischen Zugbandes 18 entsprechen kann. Die Länge der schlitzartigen weiteren Ausnehmung 26 kann jeweils variieren, so dass die verschiedenen weiteren Ausnehmungen 26 jeweils unterschiedlich lang sein können. Die weiteren Ausnehmungen 26 können zudem leicht gekrümmt sein, wie dies die Figuren 2A bis 2E andeutungsweise erkennen lassen, verlaufen mit ihrer Hauptrichtung jedoch in etwa parallel zur Längserstreckungsrichtung des weiteren Zugbandes 18. Außerdem kann die Breite der jeweiligen weiteren Ausnehmung 26 in etwa zwischen 10% und 50% der Breite des weiteren Zugbandes 18 an der Stelle der Ausnehmung 26 betragen. Das Zugband 18 kann wahlweise nur eine Ausnehmung 26 über seiner Breite aufweisen. Es können gemäß Figuren 2A bis 2E auch jeweils paarweise nebeneinander angeordnete weitere Ausnehmungen 26 im weiteren Zugband 18 befinden, die über die Breite des Zugbandes 18 so voneinander beabstandet sind, dass drei etwa gleichbreite Streifen an den Stellen der Ausnehmungen 26 gebildet sind.

Die Ausnehmungen 24, 26 des wenigstens einen weiteren Zugbandes 18 und des wenigstens einen Zugbandes 16 haben jeweils eine längliche und geschwungene Form, wahlweise auch einen geradlinig gestreckten Längsverlauf.

Gemäß der Figuren 3A und 3B umfasst das wenigstens eine elastische Zugband 16, 16' an einer bestimmten Position jeweils wenigstens drei in etwa nebeinander bzw. parallel verlaufende und jeweils voneinander beabstandete Ausnehmungen 24, welche sich zumindest bereichsweise über das wenigstens eine weitere elastische Zugband 18 bzw. sichelförmig in dessen Verlauf hinein erstrecken. Die Ausnehmungen 24 sind jeweils ungefähr symmetrisch zueinander ausgebildet und verlaufen geschwungen. Auf jeder Seite des Rumpfes 20 sind jeweils wenigstens drei Ausnehmungen 24 vorgesehen.

Der medizinische Strumpf 10 ist bereichsweise durch Textil 22 gebildet. Das wenigstens eine elastische Zugband 16 und das wenigstens eine weitere elastische Zugband 18 sind auf der Außenseite des Strumpfes unter Temperatureinwirkung aufgebracht, so dass eine stoffschlüssige Verbindung zur Übertragung der von den Zugbändern 16, 18 erzeugten Zugkräfte auf den Strumpf 10 gegeben ist.

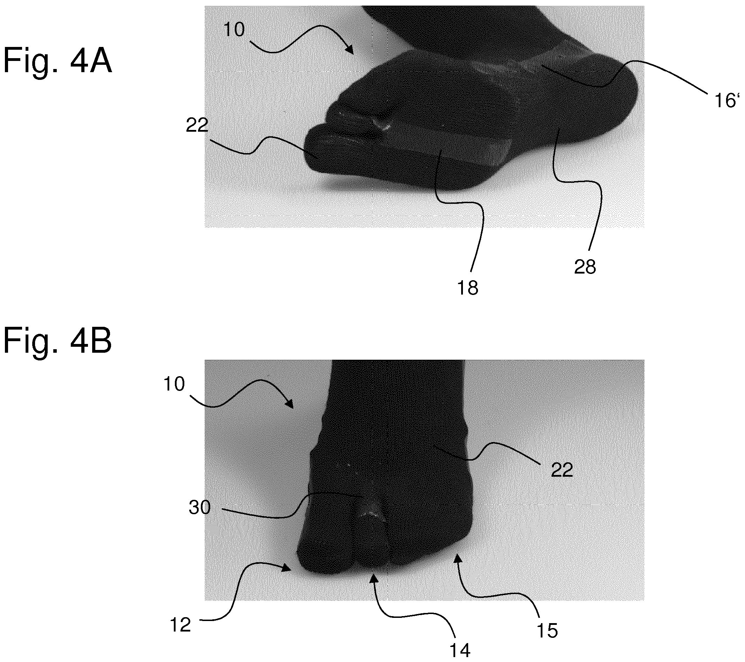

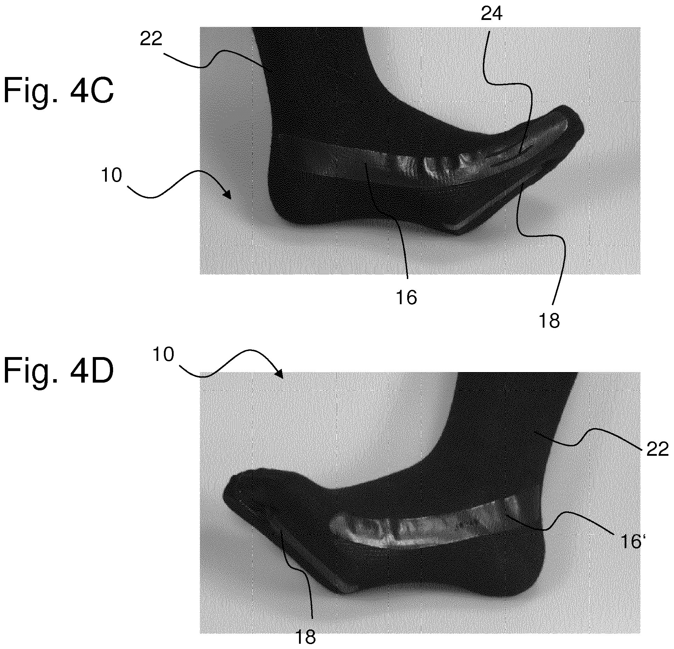

Die Figuren 4A bis 4D zeigen eine weitere Ausführungsform des medizinischen Strumpfes 10 zur Korrektur von Fuß- und/oder Beinfehlstellungen, insbesondere von Hallux Valgus und Hammerzehen, wobei die Wirkungsweise des mindestens einen elastischen Zugmittels, gebildet durch das wenigstens eine elastische Zugband 16, 16' wie bei den Ausführungsformen gemäß der Figuren 1 bis 3B entspricht.

Der medizinische Strumpf 10 umfasst wenigstens drei getrennte Kammern. Die erste Kammer 12 nimmt die Großzehe, die zweite Kammer 14 nimmt die zweite Zehe und die dritte Kammer 15 nimmt die weiteren Zehen auf. Der medizinische Strumpf 10 umfasst mindestens ein elastisches Zugmittel in Form von wenigstens einem elastischen Zugband 16, 16', welches medial und lateral angeordnet ist. Das wenigstens eine elastische Zugband 16 erstreckt sich medial längsgerichtet zumindest abschnittsweise entlang eines in die wenigstens zwei Kammern mündenden Rumpfbereichs 20 des Strumpfes. Das medial angeordnete wenigstens eine elastische Zugband 16 läuft vom Bereich der Ferse bis zur Großzehe, so dass das wenigstens eine elastische Zugband 16 zur Beaufschlagung der ersten Kammer 12 gegenüber der wenigstens einen zweiten Kammer 14 mit einer Zugkraft ausgebildet ist. Die Zugkraft sorgt dafür, dass die Großzehe in seine natürliche Position gebracht wird. Der medizinische Strumpf 10 umfasst wenigstens ein lateral angeordnetes elastische Zugband 16', welches als Gegenlager gegenüber dem medial angeordneten wenigstens einem elastischen Zugband 16 gebildet ist und sich von der Ferse bis zum Außenballen des Strumpfes erstreckt.

Das wenigstens eine elastische Zugband 16 umfasst an einer bestimmten Position wenigstens zwei Ausnehmungen 24, welche zumindest teilweise längsgerichtet entlang der Längserstreckungsrichtung des wenigstens einen elastischen Zugbandes 16 verlaufen.

Das mindestens eine Zugmittel umfasst wenigstens ein weiteres elastisches Zugband 18. Das wenigstens eine weitere elastische Zugband 18 ist auf der Sohle 28 des Strumpfes 10 angeordnet. Das wenigstens eine weitere elastische Zugband 18 erstreckt sich etwa mittig von der Sohle 28 des Strumpfes 10 zumindest näherungsweise bis zur zweiten getrennten Kammer 14. Im Bereich der zweiten Kammer 14 gabelt sich das wenigstens eine weitere elastische Zugband 18 in zumindest näherungsweise zwei gleichbreite Zugbandstreifen 30 auf. Die zumindest zwei Bandstreifen 30 umgreifen die wenigstens eine zweite Kammer 14 zumindest vollständig, wobei sich jeweils die Enden der Zugbandstreifen 30 überlappen. Mittels des wenigstens einen weiteren elastischen Zugbandes 18 wird die zweite Kammer 14 mit einer vertikal nach unten gerichteten Zugkraft beaufschlagt. Fußfehlfehlstellungen, wie beispielsweise Hammerzehen, können auf diese Weise behandelt werden.

Die jeweils zu beaufschlagende Kraft, sei es durch das wenigstens eine elastische Zugband 16, als auch über das wenigstens eine weitere elastische Zugband 18, wird über die Schichtanzahl eingestellt. Die Zugkraft ist umso größer, aus je mehr Schichten das wenigstens eine elastische Zugband und/oder wenigstens eine elastische Band gebildet sind und umgekehrt.

Das wenigstens eine elastische Zugband 16 ist in Richtung der wenigstens zwei Kammern verjüngt ausgebildet, wobei dadurch die Zugkraft auf die in der ersten Kammer 12 befindliche Großzehe dosiert wird. Das wenigstens eine weitere elastische Zugband 18 ist in Richtung der Ferse verjüngt ausgebildet, wobei dadurch die Zugkraft auf die in der zweiten Kammer 12 befindliche Zehe dosiert wird.

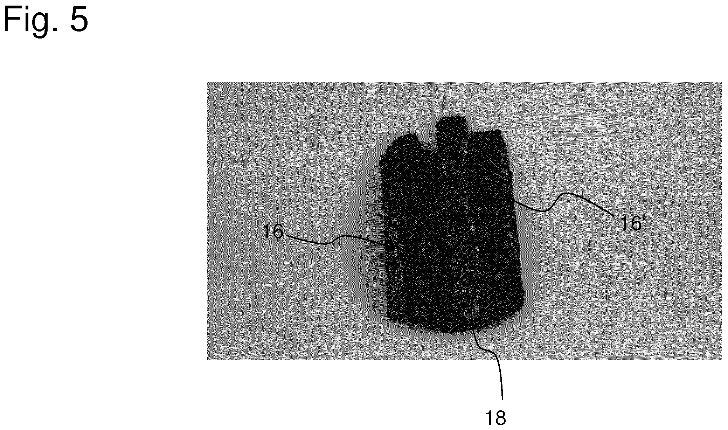

Die Fig. 5 zeigt ein weiteres Ausführungsbeispiel des medizinischen Strumpfes 10. Die Fig. 5 unterscheidet sich gegenüber das Ausführungsbeispiel der Figuren 4A bis 4D darin, dass das wenigstens eine weitere elastische Zugband 18 von der Sohle 28 bis zur Ferse des Strumpfes 10 verläuft.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezuaszeichenliste

- 10: Medizinischer Strumpf
- 12: Erste Kammer
- 14: Zweite Kammer
- 15: Dritte Kammer
- 16: Medial angeordnetes elastisches Zugband
- 16': Lateral angeordnetes elastisches Zugband
- 18: Weiteres elastisches Zugband
- 20: Rumpfbereich
- 22: Textil
- 24: Ausnehmung
- 26: Weitere Ausnehmung
- 28: Sohle
- 30: Zugbandstreifen

## Patentansprüche

1. Medizinischer Strumpf (10), umfassend:
- wenigstens zwei getrennte Kammern, wobei eine erste der wenigstens zwei getrennten Kammern zur Aufnahme einer Großzehe und wenigstens eine zweite Kammer zur Aufnahme mindestens einer weiteren Zehe eines den Strumpf (10) tragenden menschlichen Fußes ausgebildet ist, sowie
- mindestens ein elastisches Zugmittel zur Erzeugung einer definierten Zugkraft, welches mindestens eine elastische Zugmittel wenigstens ein elastisches Zugband (16) umfasst, welches sich lateral und medial zumindest abschnittsweise entlang eines in die wenigstens zwei Kammern mündenden Rumpfbereiches (20) des Strumpfes (10) erstreckt, **dadurch gekennzeichnet, dass** das wenigstens eine elastische Zugband (16) sich medial vom Bereich von den wenigstens zwei Kammern und damit vom Vorderfuß entlang des Mittelfußes bis zur Ferse erstreckt und wobei das lateral angeordnete wenigstens eine elastische Zugband (16') als Gegenlager für das medial angeordnete wenigstens eine elastische Zugband (16) dient,
- wobei das wenigstens eine elastische Zugband (16) an mindestens einer definierten Position wenigstens eine Ausnehmung (24) besitzt und/oder über mindestens einen Abschnitt seiner Längserstreckung durch wenigstens eine schlitzartige Ausnehmung (24, 26) in eine Querrichtung unterteilt ist, und
- wobei das mindestens eine elastische Zugmittel wenigstens ein weiteres elastisches Zugband (18) umfasst, welches wenigstens eine weitere elastische Zugband (18) zumindest abschnittsweise um den Rumpfbereich (20) geführt ist, und
- wobei das wenigstens eine weitere elastische Zugband (18) an mindestens einer bestimmten Position wenigstens eine weitere Ausnehmung (26) besitzt, welche wenigstens eine weitere Ausnehmung (26) sich zumindest abschnittsweise entlang der Längserstreckungsrichtung des wenigstens einen weiteren elastischen Zugbandes (18) erstreckt.

2. Medizinischer Strumpf nach Anspruch 1, bei welchem sich die wenigstens eine Ausnehmung (24) zumindest abschnittsweise entlang der Längserstreckungsrichtung des wenigstens einen elastischen Zugbandes (16) erstreckt und das Zugband (16) dadurch zumindest abschnittsweise in wenigstens zwei voneinander separierte Segmente oder Streifen (30) unterteilt, die jeweils wiederum elastisch sind.

3. Medizinischer Strumpf nach Anspruch 1 oder 2, bei welchem sich das wenigstens eine elastische Zugband (16) in Richtung der wenigstens zwei Kammern in seiner Breite verjüngt.

4. Medizinischer Strumpf nach einem der vorherigen Ansprüche, bei welchem das wenigstens eine elastische und sich längsgerichtet zumindest abschnittsweise entlang des in die wenigstens zwei Kammern mündenden Rumpfbereichs (20) erstreckende Zugband (16) die wenigstens zwei getrennten Kammern zumindest näherungsweise erreicht.

5. Medizinischer Strumpf nach einem der vorherigen Ansprüche, bei welchem das wenigstens eine elastische Zugband (16) und das wenigstens eine weitere elastische Zugband (18) schräg zueinander orientiert sind und/oder ineinander übergehen und/oder sich an einer Stelle kreuzen.

6. Medizinischer Strumpf nach einem der Ansprüche 1 bis 4, bei welchem das wenigstens eine weitere elastische Zugband (18) das wenigstens eine elastische Zugband (16) zumindest näherungsweise erreicht oder bei welchem das wenigstens eine weitere elastische Zugband (18) und das wenigstens eine elastische Zugband (16) ineinander übergehen und/oder sich an einer Stelle kreuzen.

7. Medizinischer Strumpf nach einem der vorherigen Ansprüche, wobei das wenigstens eine weitere elastische Zugband (18) an mindestens einer bestimmten Position wenigstens eine weitere Ausnehmung (26) besitzt, welche wenigstens eine weitere Ausnehmung (26) sich zumindest abschnittsweise entlang der Längserstreckungsrichtung des wenigstens einen weiteren elastischen Zugbandes (18) erstreckt und das wenigstens eine weitere elastische Zugband (18) dadurch zumindest abschnittsweise in wenigstens zwei voneinander separierte Segmente oder Streifen (30) unterteilt, die jeweils wiederum elastisch sind.

8. Medizinischer Strumpf nach einem der vorherigen Ansprüche, bei welchem sich die wenigstens eine Ausnehmung (24) des wenigstens einen elastischen Zugbandes (16) zumindest abschnittsweise in das wenigstens eine weitere elastische Zugband (18) erstreckt.

9. Medizinischer Strumpf nach einem der Ansprüche 1 bis 4, bei welchem das mindestens eine elastische Zugmittel wenigstens ein weiteres elastisches Zugband (18) umfasst, welches sich zumindest abschnittsweise entlang einer in die wenigstens zwei Kammern mündender Sohle (28) des Strumpfes (10) erstreckt und zumindest eine Kammer der wenigstens zwei getrennten Kammern zumindest abschnittsweise umgreift.

10. Medizinischer Strumpf nach Anspruch 9, bei welchem sich das wenigstens eine elastische Zugband (16) im Bereich von einer der wenigstens zwei getrennten Kammern in zumindest zwei elastische Zugbandstreifen (30) aufteilt.

11. Medizinischer Strumpf nach Anspruch 9 oder 10, bei welchem sich Enden der zumindest zwei elastischen Zugbandstreifen (30) zumindest näherungsweise erreichen oder überlappen.

12. Medizinischer Strumpf nach einem der vorherigen Ansprüche, bei welchem die definierte Zugkraft über die wenigstens eine Ausnehmung (24) und/oder über die wenigstens eine weitere Ausnehmung (26) einstellbar und/oder dosierbar ist.

13. Medizinischer Strumpf nach einem der vorherigen Ansprüche, welcher zumindest bereichsweise durch Textil (22) gebildet ist, wobei das mindestens ein Zugmittel auf das Textil (22) aufgebracht und/oder in das Textil (22) eingearbeitet ist.

14. Medizinischer Strumpf nach einem der vorherigen Ansprüche, bei welchem das wenigstens eine elastische Zugband (16) und/oder das wenigstens eine weitere elastische Zugband (18) durch ein thermoplastisches Kunststoffmaterial gebildet sind.

## Claims

1. A medical stocking (10), comprising:
- at least two separate chambers, wherein a first of the at least two separate chambers is designed to accommodate a big toe, and at least one second chamber is designed to accommodate at least one further toe of a human foot wearing the stocking (10), as well as
- at least one elastic pulling means to produce a defined pulling force, which at least one elastic pulling means comprises at least one elastic pulling strap (16), which extends laterally and medially at least in some sections along a body area (20) of the stocking (10), which body area (20) leads into the at least two chambers, **characterised in that** the at least one elastic pulling strap (16) extends medially from the area of the at least two chambers and thus from the forefoot along the mid-foot to the heel, and wherein the laterally arranged at least one elastic pulling strap (16') serves as counter bearing for the medially arranged at least one elastic pulling strap (16),
- wherein the at least one elastic pulling strap (16) has at least one recess (24) in at least one defined position and/or is subdivided in at least one section of its longitudinal extension in a transverse direction by at least one slit-like recess (24, 26), and
- wherein the at least one elastic pulling means comprises at least one further elastic pulling strap (18), which at least one further elastic pulling strap (18) is guided at least in some sections around the body area (20), and
- wherein the at least one further elastic pulling strap (18) has at least one further recess (26) in at least one determined position, which at least one further recess (26) extends at least in some sections along the longitudinal extension direction of the at least one further elastic pulling strap (18).

2. The medical stocking according to claim 1, in which the at least one recess (24) extends at least in some sections along the longitudinal extension direction of the at least one elastic pulling strap (16), and the pulling strap (16) is thereby at least in some sections subdivided into at least two segments or strips (30), which are separate from each other and which each in turn are elastic.

3. The medical stocking according to claim 1 or 2, in which the at least one elastic pulling strap (16) tapers in its width toward the at least two chambers.

4. The medical stocking according to one of the previous claims, in which the at least one elastic pulling strap (16) extending lengthwise at least in some sections along the body area (20), which body area (20) leads into the at least two chambers, at least approximately reaches the at least two separate chambers.

5. The medical stocking according to one of the previous claims, in which the at least one elastic pulling strap (16) and the at least one further elastic pulling strap (18) are oriented obliquely to each other and/or merge with each other and/or intersect in one place.

6. The medical stocking according to one of the claims 1 to 4, in which the at least one further elastic pulling strap (18) at least approximately reaches the at least one elastic pulling strap (16), or in which the at least one further elastic pulling strap (18) and the at least one elastic pulling strap (16) merge with each other and/or intersect in one place.

7. The medical stocking according to one of the previous claims, wherein the at least one further elastic pulling strap (18) has at least one further recess (26) in at least one determined position, which at least one further recess (26) extends at least in some sections along the longitudinal extension direction of the at least one further elastic pulling strap (18), and the at least one further elastic pulling strap (18) is thereby at least in some sections subdivided into at least two segments or strips (30), which are separate from each other and which each in turn are elastic.

8. The medical stocking according to one of the previous claims, in which the at least one recess (24) of the at least one elastic pulling strap (16) extends at least in some sections into the at least one further elastic pulling strap (18).

9. The medical stocking according to one of the claims 1 to 4, in which the at least one elastic pulling means comprises at least one further elastic pulling strap (18), which extends at least in some sections along a sole (28) of the stocking (10), which sole (22) leads into the at least two chambers and encompasses at least in some sections at least one chamber of the at least two separate chambers.

10. The medical stocking according to claim 9, in which the at least one elastic pulling strap (16) divides into at least two elastic pulling strap strips (30) in the area of one of the at least two separate chambers.

11. The medical stocking according to claim 9 or 10, in which ends of the at least two elastic pulling strap strips (30) at least approximately reach to each other or overlap.

12. The medical stocking according to one of the previous claims, in which the defined pulling force is adjustable and/or adaptable by way of the at least one recess (24) and/or by way of the at least one further recess (26).

13. The medical stocking according to one of the previous claims, which is at least in some areas formed by textile material (22), wherein the at least one pulling means is applied onto the textile material (22) or worked into the textile material (22).

14. The medical stocking according to one of the previous claims, in which the at least one elastic pulling strap (16) and/or the at least one further elastic pulling strap (18) are formed by a thermoplastic synthetic material.

## Revendications

1. Bas médical (10), comprenant :
- au moins deux chambres séparées, une première desdites au moins deux chambres séparées étant réalisée pour loger un gros orteil, et au moins une deuxième chambre pour loger au moins un autre orteil d'un pied humain portant ledit bas (10), ainsi qu'
- au moins un moyen de traction élastique destiné à générer une force de traction déterminée, lequel au moins un moyen de traction élastique comprend au moins une sangle de traction (16) élastique qui s'étend latéralement et médialement au moins partiellement le long d'une zone tronc (20) du bas (10) débouchant dans lesdites au moins deux chambres, **caractérisé en ce que** ladite au moins une sangle de traction (16) élastique s'étend médialement à la zone à partir desdites au moins deux chambres, et ainsi de l'avant-pied, le long du métatarse jusqu'au talon, et ladite au moins une sangle de traction (16') élastique agencée latéralement sert de contre-appui à ladite au moins une sangle de traction (16) élastique agencée médialement,
- ladite au moins une sangle de traction (16) élastique possédant, à au moins une position déterminée, au moins un évidement (24) et/ou étant, sur au moins une portion de son étirement longitudinal, divisée par au moins un évidement (24, 26) en forme de fente dans un sens perpendiculaire, et
- ledit au moins un moyen de traction élastique comprenant au moins une autre sangle de traction (18) élastique, laquelle au moins une autre sangle de traction (18) élastique est dirigée au moins partiellement autour de la zone tronc (20), et
- ladite au moins une autre sangle de traction (18) élastique possédant à au moins une position déterminée au moins un autre évidement (26), lequel au moins un autre évidement (26) s'étend au moins partiellement le long du sens d'étirement longitudinal de ladite au moins une autre sangle de traction (18) élastique.

2. Bas médical selon la revendication 1, dans lequel ledit au moins un évidement (24) s'étend au moins partiellement le long du sens d'étirement longitudinal de ladite au moins une sangle de traction (16) élastique et divise ainsi ladite sangle de traction (16) au moins partiellement en au moins deux segments ou bandes (30) séparé(e)s l'un(e) de l'autre qui sont, quant à eux/elles, chacun(e) élastique.

3. Bas médical selon la revendication 1 ou 2, dans lequel ladite au moins une sangle de traction (16) élastique se rétrécit dans sa largeur en direction desdites au moins deux chambres.

4. Bas médical selon l'une quelconque des revendications précédentes, dans lequel ladite au moins une sangle de traction (16), qui est élastique et s'étire, dans le sens de la longueur, au moins partiellement le long de la zone tronc (20) débouchant dans lesdites au moins deux chambres, atteint au moins approximativement lesdites au moins deux chambres séparées.

5. Bas médical selon l'une quelconque des revendications précédentes, dans lequel ladite au moins une sangle de traction (16) élastique et ladite au moins une autre sangle de traction (18) élastique sont orientées à la transversale l'une de l'autre et/ou se prolongent l'une dans l'autre et/ou se croisent à un endroit.

6. Bas médical selon l'une quelconque des revendications 1 à 4, dans lequel ladite au moins une autre sangle de traction (18) élastique atteint au moins approximativement ladite au moins une sangle de traction (16) élastique, ou dans lequel ladite au moins une autre sangle de traction (18) élastique et ladite au moins une sangle de traction (16) élastique se prolongent l'une dans l'autre et/ou se croisent à un endroit.

7. Bas médical selon l'une quelconque des revendications précédentes, ladite au moins une autre sangle de traction (18) élastique comprenant à au moins une position déterminée au moins un autre évidement (26), lequel au moins un autre évidement (26) s'étend au moins partiellement le long du sens d'étirement longitudinal de ladite au moins une autre sangle de traction (18) élastique et divise ainsi au moins partiellement ladite au moins une autre sangle de traction (18) élastique en au moins deux segments ou bandes (30) séparé(e)s l'un(e) de l'autre qui sont, à leur tour, chacun(e) élastique.

8. Bas médical selon l'une quelconque des revendications précédentes, dans lequel ledit au moins un évidement (24) de ladite au moins une sangle de traction (16) élastique s'étend au moins partiellement dans ladite au moins une autre sangle de traction (18) élastique.

9. Bas médical selon l'une quelconque des revendications 1 à 4, dans lequel ledit au moins un moyen de traction élastique comprend au moins une autre sangle de traction (18) élastique qui s'étend au moins partiellement le long d'une semelle (28) du bas (10) débouchant dans lesdites au moins deux chambres, et enserre au moins partiellement au moins une chambre desdites au moins deux chambres séparées.

10. Bas médical selon la revendication 9, dans lequel ladite au moins une sangle de traction (16) élastique se divise, au niveau d'une desdites au moins deux chambres séparées, en au moins deux bandes de sangle de traction (30) élastiques.

11. Bas médical selon la revendication 9 ou 10, dans lequel des extrémités desdites au moins deux bandes de sangle de traction (30) élastiques s'atteignent au moins approximativement ou se chevauchent.

12. Bas médical selon l'une quelconque des revendications précédentes, dans lequel la force de traction déterminée peut être adaptée et/ou dosée par le biais dudit au moins un évidement (24) et/ou par le biais dudit au moins un autre évidement (26).

13. Bas médical selon l'une quelconque des revendications précédentes, lequel est constitué au moins pour certaines zones de matière textile (22), ledit au moins un moyen de traction étant appliqué sur la matière textile (22) et/ou étant intégré dans ladite matière textile (22).

14. Bas médical selon l'une quelconque des revendications précédentes, dans lequel ladite au moins une sangle de traction (16) élastique et/ou ladite au moins une autre sangle de traction (18) élastique sont constituées d'une matière thermoplastique.
